# EUROPEAN PATENT APPLICATION

(11) **EP 2 927 349 A1**
(43) Date of publication of application: **07.10.2015**
(21) Application number: 13858543.5
(22) Date of filing: 29.11.2013
(51) Int. Cl.: C25B 9/00, C25B 1/04, A61M 37/00

(54) **DEVICE FOR SUPPLYING HIGHLY CONCENTRATED HYDROGEN GAS FOR BIOLOGICAL APPLICATIONS**

(30) Priority: 30.11.2012 JP 2012263155
(71) Applicant: MIZ Co., Ltd., Fujisawa-shi Kanagawa 251-0871 (JP)
(72) Inventor: SATOH, Fumitake, Fujisawa-shi Kanagawa 251-0871 (JP); KUROKAWA, Ryousuke, Fujisawa-shi Kanagawa 251-0871 (JP); SATOH, Bunpei, Fujisawa-shi Kanagawa 251-0871 (JP)
(74) Representative: Ter Meer Steinmeister & Partner
(86) International application number: PCT/JP2013/082169
(87) International publication number: WO 2014/084348

(57) **Abstract**

An apparatus includes: an electrolytic cell (6) having an electrolytic chamber (3) to which subject raw water (2) to be electrolyzed is introduced, at least one membrane (13) that partitions inside and outside of the electrolytic chamber, and at least a pair of electrode plates (4, 5) provided in the inside and the outside of the electrolytic chamber so as to sandwich the membrane, the electrode plate in the outside of the electrolytic chamber being provided to be in contact with the membrane; a direct-current power source (14) that applies a direct-current voltage to the pair of electrode plates; and a diluent gas supply means (10, 11) for supplying a diluent gas for diluting hydrogen gas generated from an electrode plate of the pair of electrode plates that is to be the cathode, wherein at least a part of a gas phase portion (7) formed in the electrolytic chamber is covered with a hydrogen gas permeable membrane (8), the apparatus blows diluent gas supplied using the diluent gas supply means to the cathode or a cathode water surface thereby to constantly maintain a hydrogen gas concentration in a position separated by 7 cm from the cathode or the cathode water surface at lower than 18.3 vol% during electrolysis so that mixed gas comprising the hydrogen gas and the diluent gas is supplied to a living organism, the mixed gas having a hydrogen gas concentration of 0.1 vol% or higher and lower than 18.3 vol%.

## Description

### [Technical Field]

The present invention relates to an apparatus for supplying high-concentration hydrogen gas for a living organism.

### [Background Art]

There is known an apparatus for supplying hydrogen gas for a living organism configured such that an air mixer is attached to a part of a conduit pipe from a hydrogen gas generator to a nasal cavity cannula and the concentration of hydrogen gas for supply can be arbitrarily set (Patent Document 1).

### [Prior Art Document]

### [Patent Document]

[Patent Document 1] JP 2009-5881 A

### [Summary of Invention]

### [Problems to be solved by Invention]

According to the above apparatus for supplying hydrogen gas for a living organism, however, hydrogen gas of a high concentration passes through the conduit pipe from the hydrogen gas generator to the air mixer because the air mixer is attached to a part of the conduit pipe to the nasal cavity cannula to mix air with the generated hydrogen gas. Therefore, the apparatus cannot be used safely in medical practice or at home.

Problems to be solved by the present invention include providing an apparatus for supplying high-concentration hydrogen gas for a living organism by which hydrogen gas having health benefits can be used safely in medical practice or at home.

### [Means for solving problems]

The present invention solves the above problems through providing an apparatus for supplying high-concentration hydrogen gas for a living organism. The apparatus is configured to dilute hydrogen gas, which is generated in an electrolytic chamber or in a side chamber via electrolysis, in the vicinity of a cathode or a cathode water surface using a diluent gas supplier, or to discharge a part of the generated hydrogen gas outside the chambers and dilute hydrogen gas in the chambers using a diluent gas supplier or a suction pump. The apparatus can thereby maintain the hydrogen gas concentration at lower than 18.3 vol%, which is the lower limit of detonation, in all the course from a time when the hydrogen gas is generated to a time when the hydrogen gas is supplied to a living organism.

### [Effect of Invention]

According to the present invention, hydrogen gas having health benefits can be used safely in medical practice or at home.

### [Brief Description of Drawings]

[Fig. 1] FIG. 1 is a diagram illustrating an apparatus for supplying hydrogen gas for a living organism according to an embodiment of the present invention.
[Fig. 2] FIG. 2 is a diagram illustrating an apparatus for supplying hydrogen gas for a living organism according to another embodiment of the present invention.
[Fig. 3] FIG. 3 is a diagram illustrating an apparatus for supplying hydrogen gas for a living organism according to still another embodiment of the present invention.
[Fig. 4] FIG. 4 is a diagram illustrating an apparatus for supplying hydrogen gas for a living organism according to yet another embodiment of the present invention.
[Fig. 5] FIG. 5 is a diagram illustrating an apparatus for supplying hydrogen gas for a living organism according to a further embodiment of the present invention.
[Fig. 6] FIG. 6 is a diagram illustrating an apparatus for supplying hydrogen gas for a living organism according to a still further embodiment of the present invention.
[Fig. 7] FIG. 7 is a diagram illustrating an apparatus for supplying hydrogen gas for a living organism according to a yet further embodiment of the present invention.

### [Mode(s) for Carrying out the Invention]

The apparatus for supplying hydrogen gas for a living organism according to the present invention is an apparatus that supplies hydrogen gas to a living organism mainly for the purpose of health maintenance and/or functional maintenance of living organisms (including cells and organs), disease improvement and/or functional improvement, or health check and/or functional measurement. Examples of supply way for hydrogen gas may include, but are not limited to, supply by way of inhalation from the nasal cavity and/or mouth cavity; supply by way of exposure of and/or blowing to the skin or organ; supply by way of exposure of and/or blowing to a living organism applicable liquid, such as liquid drug and organ storage liquid, which is assumed to be applied to a living organism; and supply by way of diffusion from the outside of a container or circuit which is provided with a living organism.

In the present invention, by introducing subject raw water into an electrolytic chamber or a side chamber provided outside the electrolytic chamber and applying a direct-current voltage from a direct-current power source, the subject raw water is electrolyzed to generate hydrogen gas at the cathode side.

The hydrogen gas generated at the cathode side via electrolysis transfers to a gas phase portion in the electrolytic chamber or the side chamber. Here, when the electrolytic chamber or the side chamber is closed such as by a cover to avoid diffusion of the hydrogen gas into the air, the hydrogen concentration in the gas phase portion increases as time passes and will exceed 18.3 vol%, which is the lower limit of detonation of hydrogen gas. On the other hand, even when the electrolytic chamber or the side chamber is not closed and the gas phase portion is opened to the air, a hydrogen gas concentration area of a high concentration will be formed, even in a local manner, at a spot either in the vicinity of the cathode or in the vicinity of the cathode water surface.

In general, it is said that hydrogen gas reacts with oxygen in the air to become detonating gas when the hydrogen concentration exceeds 4.7 vol%. According to the applicants' experiment, however, an event that such detonating gas leads to actual ignition (deflagration) may occur in most situations at a concentration much higher than 4.7 vol% which is said to be the explosion limit of hydrogen. Indeed, as long as the electrolysis is carried on, hydrogen is generated constantly from the cathode or the cathode water surface, but even if flame is moved to approach the cathode or the cathode water surface in a hydrogen concentration of 4.7 vol%, the detonation may not be audible, or may be with a considerably small sound if audible.

According to the applicants' experiment, when the hydrogen concentration exceeds 10 to 15 vol% (lower limit of deflagration), the detonation is offensive to the ear to some extent and small deflagration occurs.

However, even though a high concentration of hydrogen is intended to be obtained, it may be inappropriate to require a concentration of hydrogen higher than 18.3% (lower limit of detonation) that is a concentration at which the reaction of hydrogen and oxygen causes a shock wave to propagate as detonation around there.

According to the present invention, therefore, the hydrogen gas concentration at the point at which hydrogen of the highest concentration is generated, i.e., the hydrogen concentration in the vicinity of the cathode or the cathode water surface during electrolysis, may preferably be maintained at lower than 18.3 vol%, more preferably lower than 15.0 vol%, and further preferably lower than 4.7 vol%.

Modes for carrying out the present invention may be classified mainly into the following two types in view of the positional relationship between the electrolytic chamber and the electrode plates. That is, the two types are as follows:
1. a mode in which (at least) a pair of electrode plates is included inside the electrolytic chamber and shares the gas phase portion (the above of one electrode plate and the above of the other electrode plate are continuous) (represented by Embodiments 1, 2 and 3 below); and
2. a mode in which a membrane partitions the inside and the outside of the electrolytic chamber, and one of (at least) a pair of electrode plates is provided inside the electrolytic chamber while the other electrode plate is provided outside to be in contact with the membrane, so that the electrode plates do not share a gas phase portion (the above of one electrode plate and the above of the other electrode plate are separated) (represented by Embodiments 4 and 5 below).

From this viewpoint, some possible embodiments according to the present invention will be described hereinafter.

### (Embodiment 1)

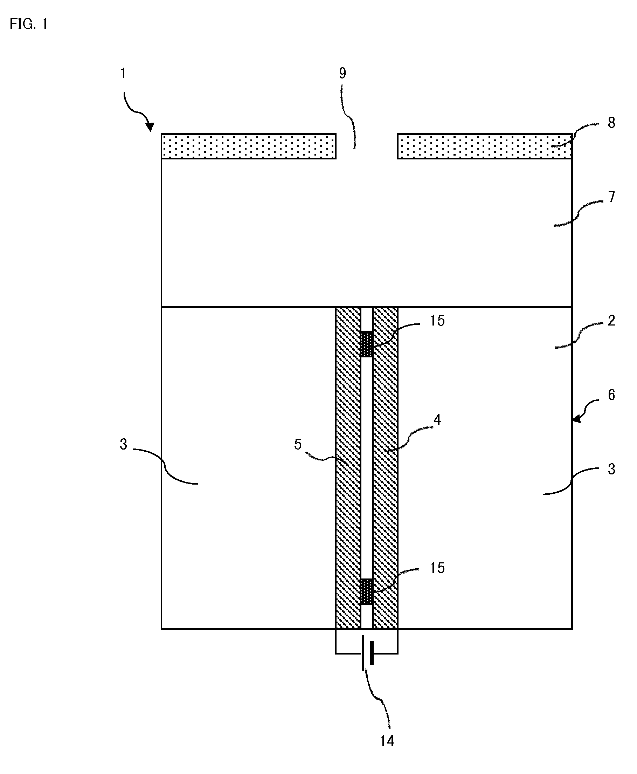

As shown in FIG. 1, an apparatus 1 for supplying high-concentration hydrogen gas for a living organism according to an embodiment of the present invention includes an electrolytic cell 6 that has: an electrolytic chamber 3 to which subject raw water 2 is introduced; and at least a pair of electrode plates 4 and 5 included inside the electrolytic chamber 3. The electrode plates 4 and 5 are provided to be separated from each other via a distance using one or more spacers 15 or other appropriate structure. The apparatus 1 further includes a direct-current power source 14 that applies a direct-current voltage to the electrode plates 4 and 5 in the electrolytic cell 6. In an embodiment, the apparatus 1 may have a feature that a part or whole of a gas phase portion 7 in the electrolytic chamber 3 is covered with a hydrogen gas permeable membrane 8. In another embodiment, the apparatus 1 may have a feature of having an opening part 9. In a further embodiment, the apparatus 1 may have both of the features. According to the above feature or features, hydrogen gas in the gas phase portion 7 in the electrolytic chamber 3 is partially discharged outside the electrolytic chamber 3 via the hydrogen gas permeable membrane 8 or the opening part 9. Through this operation, the present invention is carried out as the apparatus 1 in which the hydrogen gas concentration in the gas phase portion 7 in the electrolytic chamber 3 can be maintained at lower than 18.3 vol% and the hydrogen gas concentration in the vicinity of the above of the hydrogen gas permeable membrane 8 or the opening part 9 can be maintained at lower than 15.0 vol%.

### (Embodiment 2)

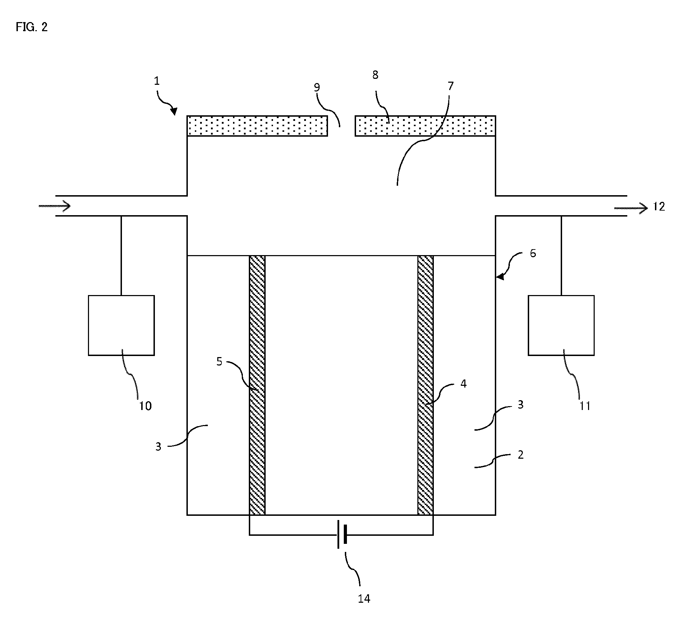

As shown in FIG. 2, an apparatus 1 for supplying high-concentration hydrogen gas for a living organism according to an embodiment of the present invention is configured such that the apparatus 1 as described in Embodiment 1 further includes at least one of a diluent gas supplier 10 or a suction pump 11 for diluting the hydrogen gas generated in the gas phase portion 7 in the electrolytic chamber 3 and for serving an appropriate flow volume of the hydrogen gas to an living organism. According to this configuration, the hydrogen gas is mixed with a diluent gas supplied from the diluent gas supplier 10 or with a diluent gas suctioned by the suction pump 11 from outside the gas phase portion 7 of the electrolytic chamber 3 via the hydrogen gas permeable membrane 8 or the opening part 9. Through this operation, the present invention is carried out as the apparatus 1 by which a living organism can be supplied with a mixed gas 12 that includes the hydrogen gas and the diluent gas and has a hydrogen concentration of lower than 15.0 vol%.

### (Embodiment 3)

An apparatus 1 for supplying high-concentration hydrogen gas for a living organism according to an embodiment of the present invention includes an electrolytic cell 6 that has: an electrolytic chamber 3 to which subject raw water 2 is introduced; and at least a pair of electrode plates 4 and 5 included inside the electrolytic chamber 3. The electrode plates 4 and 5 are provided to be separated from each other via a distance. The apparatus 1 further includes a direct-current power source 14 that applies a direct-current voltage to the electrode plates 4 and 5 in the electrolytic cell 6. The apparatus 1 is provided with a diluent gas supplier 10 for diluting the hydrogen gas generated from the electrode plate 4 or 5 that is to be a cathode, so that the diluent gas supplied from the diluent gas supplier 10 is blown to the vicinity of the electrode plate 4 or 5. Through this operation, the present invention is carried out as the apparatus 1 in which the hydrogen gas concentration in the vicinity of the electrode plate 4 or 5 can be constantly maintained at lower than 18.3 vol% during electrolysis thereby to allow a living organism to be supplied with a mixed gas 12 that includes the hydrogen gas and the diluent gas and has a hydrogen concentration of lower than the lower limit of detonation.

### (Embodiment 4)

An apparatus 1 for supplying high-concentration hydrogen gas for a living organism according to an embodiment of the present invention includes an electrolytic cell 6 that has: an electrolytic chamber 3 to which subject raw water 2 is introduced; at least one membrane 13 that partitions inside and outside of the electrolytic chamber 3; and at least a pair of electrode plates 4 and 5 provided respectively in the inside and the outside of the electrolytic chamber 3 so as to sandwich the membrane 13. The electrode plate 5 located outside the electrolytic chamber 3 or a side chamber 16 is provided to be in contact with the membrane 13. The side chamber 16 is thus provided to have the outside electrode plate 5. The apparatus 1 further includes a direct-current power source 14 that applies a direct-current voltage to the electrode plates 4 and 5 in the electrolytic cell 6. In an embodiment, the apparatus 1 may have a feature that a part or whole of a gas phase portion 7 in the electrolytic chamber 3 is covered with a hydrogen gas permeable membrane 8. In another embodiment, the apparatus 1 may have a feature of having an opening part 9. In a further embodiment, the apparatus 1 may have both of the features. According to the above feature or features, hydrogen gas in the gas phase portion 7 in the side chamber is partially discharged outside the electrolytic chamber 3 or the side chamber 16 via the hydrogen gas permeable membrane 8 or the opening part 9. Through this operation, the present invention is carried out as the apparatus 1 in which the hydrogen gas concentration in the gas phase portion 7 in the side chamber can be maintained at lower than 18.3 vol% and the hydrogen gas concentration in the vicinity of the above of the hydrogen gas permeable membrane 8 or the opening part 9 can be maintained at lower than 15.0 vol%.

### (Embodiment 5)

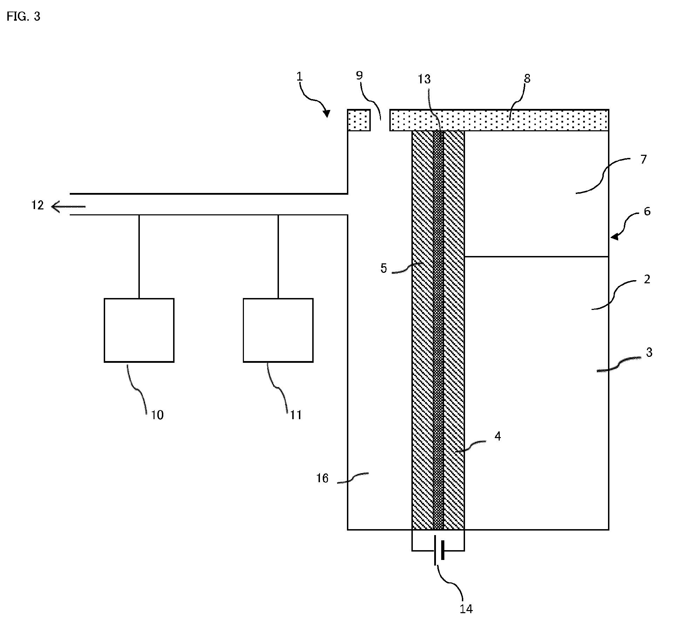

As shown in FIG. 3, an apparatus 1 for supplying high-concentration hydrogen gas for a living organism according to an embodiment of the present invention is configured such that the apparatus 1 as described in Embodiment 4 further includes at least one of a diluent gas supplier 10 or a suction pump 11 for diluting the hydrogen gas generated in the gas phase portion 7 in the side chamber and for serving an appropriate flow volume of the hydrogen gas to an living organism. According to this configuration, the hydrogen gas is mixed with a diluent gas supplied from the diluent gas supplier 10 or with a diluent gas suctioned by the suction pump 11 from outside the gas phase portion 7 of the electrolytic chamber 3 via the hydrogen gas permeable membrane 8 or the opening part 9. Through this operation, the present invention is carried out as the apparatus 1 by which a living organism can be supplied with a mixed gas 12 that includes the hydrogen gas and the diluent gas and has a hydrogen concentration of lower than 15.0 vol%.

The above is a part of possible embodiments according to the present invention, and does not exclude other possible embodiments. Examples of the electrolytic cell 6 include an electrolytic cell as described in Patent No. 3349710, etc, for example. Again, the electrolytic cell 6 has at least one membrane 13 that partitions the inside and the outside of the electrolytic chamber 3 and at least a pair of electrode plates 4 and 5 provided respectively in the inside and the outside of the electrolytic chamber 3 so as to sandwich the membrane 13. The electrode plate 5 located outside the electrolytic chamber 3 is provided to be in contact with the membrane 13.

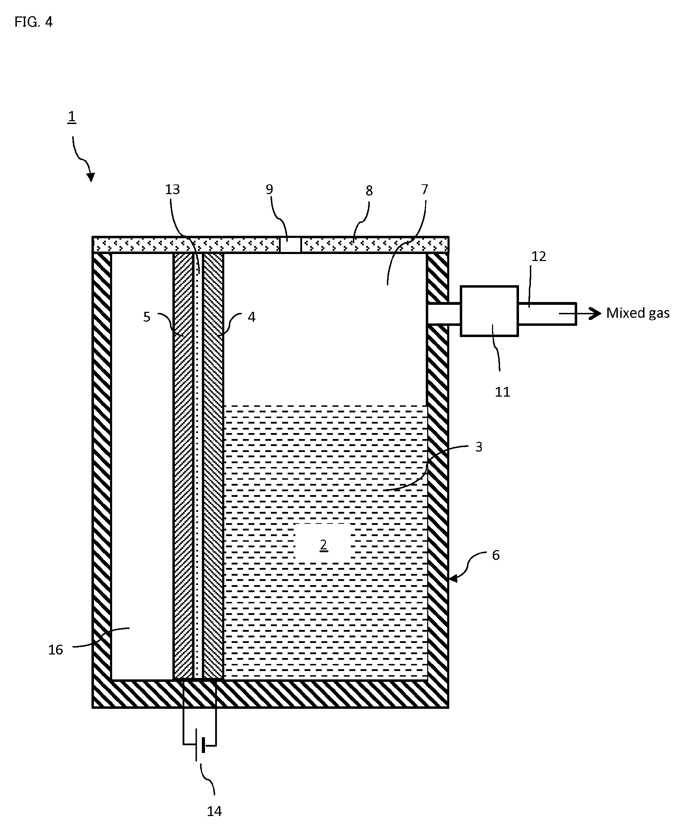

FIG. 4 to FIG. 7 each exemplify an apparatus 1 for supplying high-concentration hydrogen gas for a living organism as an embodiment according to the present invention. These embodiments are common in an aspect that the membrane 13 partitions the inside of the electrolytic cell 6 to form the electrolytic chamber 3 and the side chamber 16 which is located outside the electrolytic chamber 3 and also in an aspect that one of the pair of the electrode plates 4 and 5 is provided inside the electrolytic chamber 3 while the other electrode plate is provided outside the electrolytic chamber 3 (i.e., in the side chamber 16) so as to be in contact with the membrane 13.

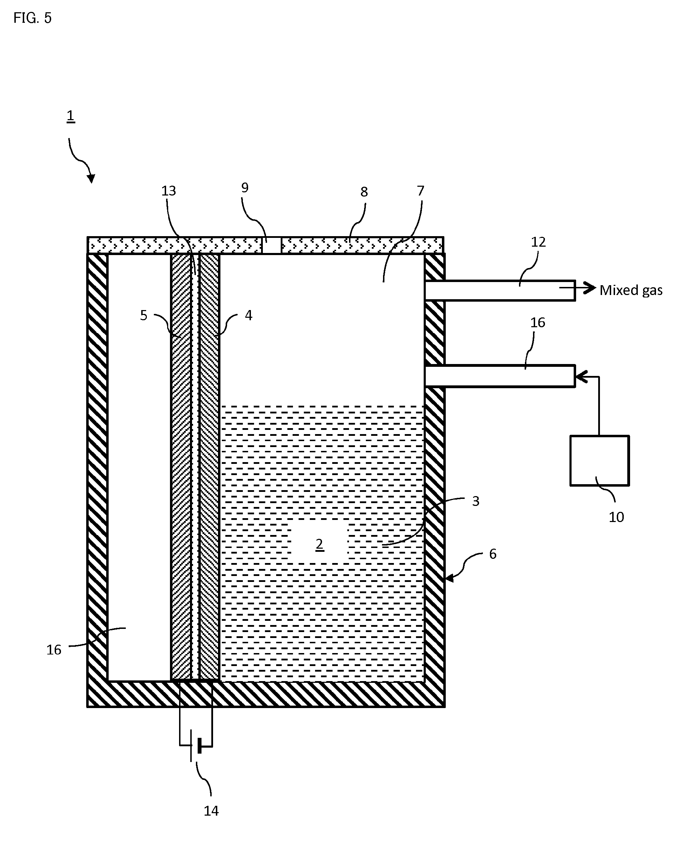

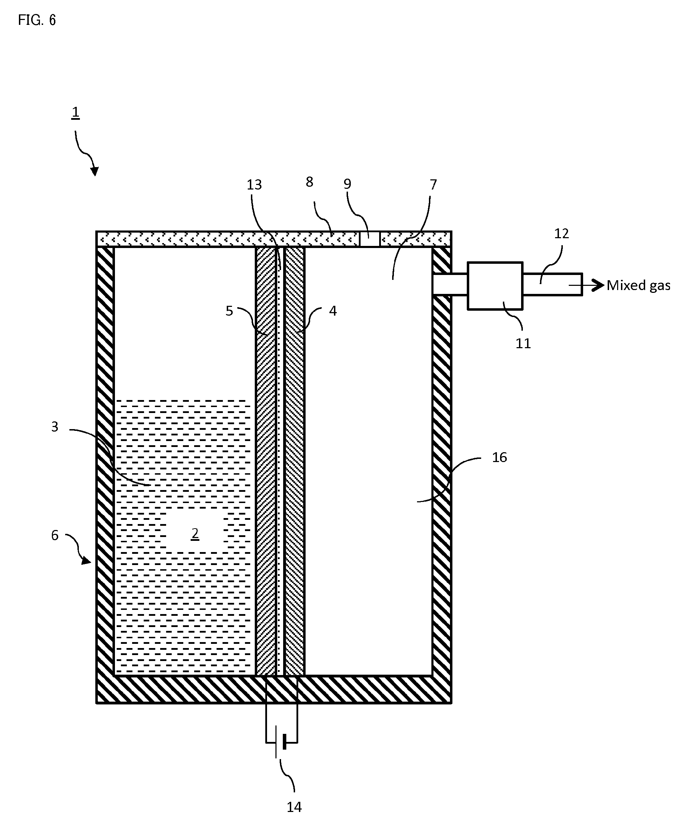

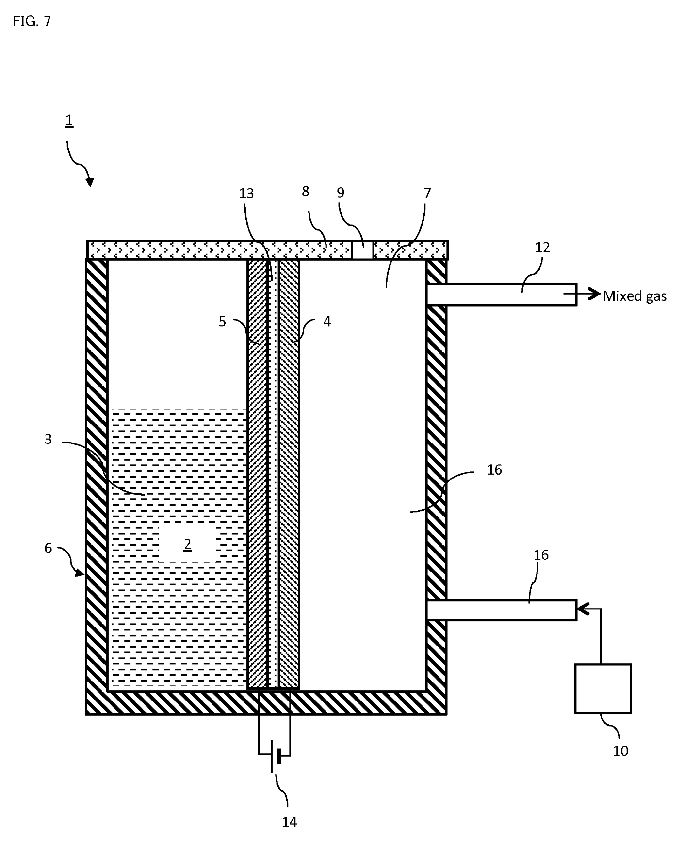

In the embodiments illustrated in FIG. 4 and FIG. 5, the electrode plate 4 provided in the electrolytic chamber 3 to which the subject raw water 2 is introduced is used as the cathode while the electrode plate 5 provided in the side chamber 16 adjacent to the electrolytic chamber 3 via the membrane 13 is used as the anode. Therefore, hydrogen gas is generated in the electrolytic chamber 3, and the gas phase portion 7 in which the hydrogen gas gathers is formed also in the electrolytic chamber 3. In contrast, in the embodiments illustrated in FIG. 6 and FIG. 7, the electrode plate 5 provided in the electrolytic chamber 3 to which the subject raw water 2 is introduced is used as the anode while the electrode plate 4 provided in the side chamber 16 adjacent to the electrolytic chamber 3 via the membrane 13 is used as the cathode. Therefore, hydrogen gas is generated in the side chamber 16, and the gas phase portion 7 in which the hydrogen gas gathers is formed also in the side chamber 16.

### (Embodiment 6)

As shown in FIG. 4, an apparatus 1 for supplying high-concentration hydrogen gas for a living organism according to an embodiment of the present invention comprises: an electrolytic cell 6; a direct-current power source 14 that applies a direct-current voltage to a pair of electrode plates 4 and 5; and a suction pump 11 that constitutes a diluent gas supply means for supplying a diluent gas for diluting hydrogen gas generated from an electrode plate of the pair of electrode plates 4 and 5 that is to be the cathode.

The electrolytic cell 6 has: an electrolytic chamber 3 to which subject raw water 2 is introduced; a membrane 13 that partitions inside and outside of the electrolytic chamber 3; and the pair of electrode plates 4 and 5 provided respectively in the inside and the outside of the electrolytic chamber 3 so as to sandwich the membrane 13. Both of the electrode plate 4 located inside the electrolytic chamber 3 and the electrode plate 5 located outside the electrolytic chamber 3 are provided to be in contact with the membrane 13. In the electrolytic cell 6 shown, one membrane 13 and one pair of electrode plates 4 and 5 are provided, but a plurality of membranes 13 and/or a plurality of pairs of electrode plates 4 and 5 may be provided. The electrode plate 4 located inside the electrolytic chamber 3 may not necessarily be provided to be in contact with the membrane 13 as long as at least the electrode plate 5 located outside the electrolytic chamber 3 is provided to be in contact with the membrane 13.

In the embodiment shown, the electrode plate 4 in the electrolytic chamber 3 to which the subject raw water is introduced is used as the cathode. Therefore, when the electrolysis of the subject raw water is performed, hydrogen gas is generated in the electrolytic chamber 3, and the generated hydrogen gas gathers in a gas phase portion 7 located at the upper part of the electrolytic chamber 3. The electrolytic cell 6 is a cylindrical container-like member having an upper opening and a bottom, and a hydrogen gas permeable membrane 8 is provided over the upper opening. That is, the hydrogen gas permeable membrane 8 is provided so as to cover at least a part of the gas phase portion 7 formed in the electrolytic chamber 3. In the embodiment shown, the hydrogen gas permeable membrane 8 extends to the outside of the electrolytic chamber 3, i.e., to the upper face of the side chamber 16, but it may be sufficient if the apparatus 1 for supplying high-concentration hydrogen gas according to the present invention covers at least a part of the gas phase portion 7 formed in the electrolytic chamber 3.

One or more opening parts 9 are formed in the hydrogen gas permeable membrane 8 which covers the gas phase portion 7 of the electrolytic chamber 3, so that gas can flow in from or flow out to the outside of the electrolytic cell 6. In view of preventing foreign substances from mixing into the generated hydrogen gas or other purposes, the opening part or parts 9 may be omitted.

A conduit pipe 12 is provided at a wall surface of the electrolytic cell 6 that corresponds to the gas phase portion 7 formed in the electrolytic chamber 3. The conduit pipe 12 introduces the hydrogen gas gathered in the gas phase portion 7 to a targeted site (such as a living organism). The conduit pipe 12 is provided with a suction pump 11. By operating this suction pump 11, gas pressure in the gas phase portion 7 is reduced, so that the outer air is introduced into the gas phase portion 7 via the hydrogen gas permeable membrane 8 and the opening part 9. The outer air dilutes the hydrogen gas in the gas phase portion 7, and the diluted hydrogen gas is then introduced to the targeted site via the conduit pipe 12. The operating condition of the suction pump 11 and the gas permeation property of the hydrogen gas permeable membrane 8 are set such that the hydrogen gas concentration in the gas phase portion 7 in the electrolytic chamber 3 is maintained at lower than 18.3 vol% and the hydrogen gas concentration in the vicinity of the above the hydrogen gas permeable membrane 8 or the opening part 9 is maintained at lower than 15.0 vol%.

### (Embodiment 7)

FIG. 5 illustrates an apparatus 1 for supplying high-concentration hydrogen gas for a living organism according to an embodiment of the present invention. This apparatus 1 has a different configuration of the diluent gas supply means from that in the apparatus 1 according to Embodiment 6 illustrated in FIG. 4, and other configurations are the same. That is, as illustrated in FIG. 5, conduit pipes 12 and 16 are provided at a wall surface of the electrolytic cell 6 that corresponds to the gas phase portion 7 formed in the electrolytic chamber 3. The conduit pipe 12 introduces the hydrogen gas gathered in the gas phase portion 7 to a targeted site (such as a living organism). The conduit pipe 16 is to forcibly supply the outer air into the gas phase portion 7. This conduit pipe 16 is provided with a diluent gas supplier 10. The diluent gas supplier 10 is to suction the outer air, such as environmental air, and introduces the outer air into the gas phase portion 7 via the conduit pipe 16. By operating the diluent gas supplier 10, gas pressure in the gas phase portion 7 increases, so that a part of the hydrogen gas in the gas phase portion 7 is discharged outside via the hydrogen gas permeable membrane 8 and the opening part 9. This allows the hydrogen gas to be diluted by the outer air in the gas phase portion 7, and the diluted hydrogen gas is then introduced to the targeted site via the conduit pipe 12. The operating condition of the diluent gas supplier 10 and the gas permeation property of the hydrogen gas permeable membrane 8 are set such that the hydrogen gas concentration in the gas phase portion 7 in the electrolytic chamber 3 is maintained at lower than 18.3 vol% and the hydrogen gas concentration in the vicinity of the above of the hydrogen gas permeable membrane 8 or the opening part 9 is maintained at lower than 15.0 vol%.

### (Embodiment 8)

As shown in FIG. 6, an apparatus 1 for supplying high-concentration hydrogen gas for a living organism according to an embodiment of the present invention comprises: an electrolytic cell 6; a direct-current power source 14 that applies a direct-current voltage to a pair of electrode plates 4 and 5; and a suction pump 11 that constitutes a diluent gas supply means for supplying a diluent gas for diluting hydrogen gas generated from an electrode plate of the pair of electrode plates 4 and 5 that is to be the cathode.

The electrolytic cell 6 has: an electrolytic chamber 3 to which subject raw water 2 is introduced; a membrane 13 that partitions inside and outside of the electrolytic chamber 3; and the pair of electrode plates 4 and 5 provided respectively in the outside and the inside of the electrolytic chamber 3 so as to sandwich the membrane 13. Both of the electrode plate 5 located inside the electrolytic chamber 3 and the electrode plate 4 located outside the electrolytic chamber 3 are provided to be in contact with the membrane 13. In the electrolytic cell 6 shown, one membrane 13 and one pair of electrode plates 4 and 5 are provided, but a plurality of membranes 13 and/or a plurality of pairs of electrode plates 4 and 5 may be provided. The electrode plate (cathode plate) 4, as shown in Figs. 4-7, may not necessarily be provided to be in contact with the membrane 13 as long as at least the electrode plate (anode plate) 5 is provided to be in contact with the membrane 13.

In the embodiment shown, the electrode plate 4 in the side chamber 16 is used as the cathode. Therefore, when the electrolysis of the subject raw water introduced in the electrolytic chamber 3 is performed, hydrogen gas is generated in the side chamber 16, and the generated hydrogen gas gathers in a gas phase portion 7 in the side chamber 16. The electrolytic cell 6 is a cylindrical container-like member having an upper opening and a bottom, and a hydrogen gas permeable membrane 8 is provided over the upper opening. That is, the hydrogen gas permeable membrane 8 is provided so as to cover at least a part of the gas phase portion 7 formed in the side chamber 16. In the embodiment shown, the hydrogen gas permeable membrane 8 extends to the outside of the side chamber 16, i.e., to the upper face of the electrolytic chamber 3, but it may be sufficient if the apparatus 1 for supplying high-concentration hydrogen gas according to the present invention covers at least a part of the gas phase portion 7 formed in the side chamber 16.

One or more opening parts 9 are formed in the hydrogen gas permeable membrane 8 which covers the gas phase portion 7 of the side chamber 16, so that gas can flow in from or flow out to the outside of the electrolytic cell 6. In view of preventing foreign substances from mixing into the generated hydrogen gas or other purposes, the opening part or parts 9 may be omitted.

A conduit pipe 12 is provided at a wall surface of the electrolytic cell 6 that corresponds to the gas phase portion 7 formed in the side chamber 16. The conduit pipe 12 introduces the hydrogen gas gathered in the gas phase portion 7 to a targeted site (such as a living organism). The conduit pipe 12 is provided with a suction pump 11. By operating this suction pump 11, gas pressure in the gas phase portion 7 is reduced, so that the outer air is introduced into the gas phase portion 7 via the hydrogen gas permeable membrane 8 and the opening part 9. The outer air dilutes the hydrogen gas in the gas phase portion 7, and the diluted hydrogen gas is then introduced to the targeted site via the conduit pipe 12. The operating condition of the suction pump 11 and the gas permeation property of the hydrogen gas permeable membrane 8 are set such that the hydrogen gas concentration in the gas phase portion 7 in the side chamber 16 is maintained at lower than 18.3 vol% and the hydrogen gas concentration in the vicinity of the above of the hydrogen gas permeable membrane 8 or the opening part 9 is maintained at lower than 15.0 vol%.

### (Embodiment 9)

FIG. 7 illustrates an apparatus 1 for supplying high-concentration hydrogen gas for a living organism according to an embodiment of the present invention. This apparatus 1 has a different configuration of the diluent gas supply means from that in the apparatus 1 according to Embodiment 8 illustrated in FIG. 6, and other configurations are the same. That is, as illustrated in FIG. 7, conduit pipes 12 and 16 are provided at a wall surface of the electrolytic cell 6 that corresponds to the gas phase portion 7 formed in the side chamber 16. The conduit pipe 12 introduces the hydrogen gas gathered in the gas phase portion 7 to a targeted site (such as a living organism). The conduit pipe 16 is to forcibly supply the outer air into the gas phase portion 7. This conduit pipe 16 is provided with a diluent gas supplier 10. The diluent gas supplier 10 is to suction the outer air, such as environmental air, and introduces the outer air into the gas phase portion 7 via the conduit pipe 16. By operating the diluent gas supplier 10, gas pressure in the gas phase portion 7 increases, so that a part of the hydrogen gas in the gas phase portion 7 is discharged outside via the hydrogen gas permeable membrane 8 and the opening part 9. This allows the hydrogen gas to be diluted by the outer air in the gas phase portion 7, and the diluted hydrogen gas is then introduced to the targeted site via the conduit pipe 12. The operating condition of the diluent gas supplier 10 and the gas permeation property of the hydrogen gas permeable membrane 8 are set such that the hydrogen gas concentration in the gas phase portion 7 in the side chamber 16 is maintained at lower than 18.3 vol% and the hydrogen gas concentration in the vicinity of the above of the hydrogen gas permeable membrane 8 or the opening part 9 is maintained at lower than 15.0 vol%.

The subject raw water as used herein is water that can generate hydrogen gas at the cathode through a process of electrolysis of the water, and examples thereof include tap water, clean water, purified water, ion-exchanged water, RO water, distilled water, and the like. The subject raw water may appropriately contain electrolytes, such as calcium ion and magnesium ion.

In order that unnecessary gas is not generated during electrolysis, it may be preferred to artificially add a water-soluble compound to pure water, such as ion-exchanged water and purified water, which does not contain solute ion, to prepare the subject raw water.

In particular, chlorine gas is known to be basically not beneficial for a living organism. It is therefore preferred that the subject raw water to be used in the present invention is subjected to a removal treatment for chlorine gas. For the same reason, in the mixed gas comprising the hydrogen gas and the diluent gas, the lower the chlorine gas concentration is, the more desirable it is. The chlorine gas concentration in the mixed gas comprising the hydrogen gas and the diluent gas may preferably 1 ppm or less, more preferably 0.5 ppm or less, and further preferably 0.1 ppm or less. Furthermore, when electrolysis is performed for water containing a water-soluble compound that releases anions when dissolved in water, such as PO4(3-), SO4(2-) and NO3(-), which have higher ionization tendency than that of hydroxide ions (it is preferred that the water itself as a solvent is preliminarily subjected to removal treatment for ions), the reaction of the hydroxide ions generating oxygen (02) while releasing electrons takes place in prior to the gasification of the anions. Therefore, unnecessary gas is less likely to be released into the gas phase portion.

The above is particularly preferred when the present invention is carried out as the "mode in which at least a pair of electrode plates is included inside the electrolytic chamber and shares the gas phase portion (the above of one electrode plate and the above of the other electrode plate are continuous)," which assumes that the hydrogen gas generated at the cathode is mixed with the gas generated at the anode.

Examples of the electrode plates to be used include, but are not limited to, those using titanium plates as base materials which are coated with a noble metal selected from the group of platinum, iridium, palladium and the like.

It is preferred that a cation exchange membrane is employed as the membrane used in the mode in which the membrane partitions the inside and the outside of the electrolytic chamber, and one of (at least) a pair of electrode plates is provided inside the electrolytic chamber while the other electrode plate is provided outside to be in contact with the membrane. In consideration of necessary factors such as the ion conductivity, physical strength, gas barrier property, chemical stability, electrochemical stability and thermal stability, there may preferably be used an all fluorine-based sulfonic acid membrane that comprises a sulfonic group as the electrolyte group. Examples of such a membrane include a membrane of Nafion (registered trademark, a DuPont product) which is a copolymer membrane of tetrafluoroethylene and perfluorovinyl ether having a sulfonic group, a membrane of Flemion (registered trademark, available from ASAHI GLASS CO., LTD.) and a membrane of Aciplex (registered trademark, available from Asahi Kasei Corporation).

In the above-described "embodiment in which the membrane partitions the inside and the outside of the electrolytic chamber, and one of at least a pair of electrode plates is provided inside the electrolytic chamber while the other electrode plate is provided outside to be in contact with the membrane," the electrode plate located inside the electrolytic chamber may be provided to be in contact with the membrane, or may also be provided to leave a small space from the membrane.

In the present invention, the gas phase portion refers to a concept including a space that is not filled with the subject raw water in the electrolytic chamber or in the side chamber. That is, the gas phase portion refers to a concept including a space that is not filled with the subject raw water at least in the chamber having the electrode plate to be the cathode, i.e., in the electrolytic chamber and/or in the side chamber.

According to the present invention, there is provided an apparatus for supplying high-concentration hydrogen gas for a living organism which can be used safely through an appropriate method of diluting such hydrogen gas staying in the gas phase portion. As described above, examples of such a method of dilution include the followings:
1. a method in which the hydrogen gas is diluted by blowing the diluent gas to the vicinity of the cathode or to the vicinity of the cathode water surface using a diluent gas supplier;
2. a method which has at least one of a feature of covering a part or whole of the gas phase portion in the electrolytic chamber or in the side chamber with the hydrogen gas permeable membrane and a feature of providing the opening part, and in which the hydrogen gas is diluted by suctioning gas (such as air) as the diluent gas from the outside of the electrolytic chamber or the side chamber to the inside of the electrolytic chamber or the side chamber using a suction (vacuum) pump; and
3. a method which has at least one of a feature of covering a part or whole of the gas phase portion in the electrolytic chamber or in the side chamber with the hydrogen gas permeable membrane and a feature of providing the opening part, and in which the hydrogen gas is diluted by discharging a part of the hydrogen gas in the electrolytic chamber or in the side chamber outside the electrolytic chamber and blowing the diluent gas to the hydrogen gas using a diluent gas supplier.

As will be appreciated, according to an aspect of the present invention, the side chamber can be understood as being functionally the same as the electrolytic chamber. Therefore, unless the both chambers are separately described or illustrated, the term "electrolytic chamber" may include the meaning of the side chamber.

Examples of the diluent gas supplier as used herein include an apparatus, such as an air pump, which can blow the diluent gas. The suction (vacuum) pump refers to a concept including a so-called vacuum pump, and is an apparatus that can dilute the hydrogen gas in the electrolytic chamber while suctioning gas (such as air) as the diluent gas from the outside of the electrolytic chamber or the side chamber to the inside of the electrolytic chamber or the side chamber.

The diluent gas refers to a concept that includes normal air and artificial air, and examples thereof include medical gas of which the oxygen concentration is adjusted and other medical gases which contain medical components such as anesthetic component.

The hydrogen gas permeable membrane may preferably be, but is not limited to, a gas membrane, such as a nonwoven fabric, which is poorly-permeable or non-permeable for water but permeable for hydrogen gas.

The vicinity of the cathode or the vicinity of the cathode water surface refers to a concept that includes a position separated by 7 cm, preferably 5 cm, more preferably 3 cm, and most preferably 1 cm, from the cathode (or the cathode water surface).

In view of the safety, it can be said that the hydrogen gas concentration in the vicinity of the above of the hydrogen gas permeable membrane or the opening part of the electrolytic chamber may be maintained preferably at lower than 15.0 vol%, more preferably at lower than 10.0 vol%, and further preferably at lower than 3.0 vol%. Here, in a similar manner as the above, the vicinity of the above of the hydrogen gas permeable membrane or the opening part of the electrolytic chamber or the side chamber refers to a concept that includes a position separated by 7 cm and preferably 3 cm from the hydrogen gas permeable membrane or the opening part, and most preferably a position in contact with the above of the hydrogen gas permeable membrane or the opening part.

In principle, the hydrogen gas concentration can be understood to increase as the measurement point comes close to the generation source of hydrogen gas. Therefore, if the measurement at the above position is difficult due to the size of the apparatus for supplying high-concentration hydrogen gas for a living organism or other reason, the measurement may be performed at a closer range than the above position but at a position as close as possible to the above position.

In the present invention, the hydrogen gas concentration in the vicinity of the cathode or the cathode water surface is measured at each of a time when 1 minute has elapsed after starting the electrolysis, a time when half an estimated electrolysis time has passed and a time when the electrolysis has been completed, and when the hydrogen gas concentration is lower than 18.3 vol% at each of the times, it is deemed that "the hydrogen gas concentration in the vicinity of the cathode or the cathode water surface is constantly maintained at lower than 18.3 vol% during electrolysis."

The present invention is an invention that relates consistently to an apparatus for supplying high-concentration hydrogen gas for a living organism. Therefore, even though the hydrogen gas concentration is to be maintained lower than 18.3 vol%, the dilution may not have to be needed beyond necessity. It is thus preferred that, unlike the handling of hydrogen gas in other industrial fields, the present invention is associated with management of the electrolytic condition and/or management of the flow volume rather than aiming to reduce the hydrogen gas concentration close to zero without limit so that the generated hydrogen gas can be safely discarded outside the system.

More specifically, in terms of the amount of air or high-concentration oxygen gas which human beings or animals inhale for 1 minute, for example, it is preferred that the diluent gas is blown at a flow volume of 1 mL/min or more, preferably 1 L/min or more, more preferably 2 L/min or more, further preferably 4 L/min or more, and particularly preferably 6 L/min or more, for example. In view of the effects to be obtained, it is preferred in the present invention that the hydrogen gas concentration in the mixed gas comprising the hydrogen gas and the diluent gas is 0.01 vol% or more, preferably 0.1 vol% or more, and more preferably 1.0 vol% or more.

Examples of a form to supply the mixed gas to a living organism include a form to inhale the mixed gas by moving the face directly to the vicinity of the cathode or cathode water surface or to the cathode chamber and a form to inhale the mixed gas from a mixed gas outlet provided at the electrolytic chamber or at the side chamber.

Moreover, an attachment such as a nasal cavity cannula may be appropriately connected to the mixed gas outlet thereby to enhance the convenience at the time of supply to a living organism and/or the stability of supply of the mixed gas.

### [Examples]

Working examples of the present invention will hereinafter be described. Unless otherwise stated in the present application, various meters used to measure various physical property values are a hydrogen gas concentration meter "XP-3140 (available from New Cosmos Electric Co., Ltd.)," an ammeter "CLAMP ON AC/DC HiTESTER 3265 (available from HIOKI E.E. CORPORATION)," and a voltmeter "CDM-2000 (available from CUSTOM corporation)."

### [Example 1]

An electrolytic cell has been prepared to be characterized by having: an electrolytic chamber to which subject raw water is introduced; the above cation exchange membrane that partitions inside and outside of the electrolytic chamber; and a pair of platinum electrodes provided in the inside and the outside of the electrolytic chamber so as to sandwich the cation exchange membrane, wherein: the electrode plate located outside the electrolytic chamber is provided to be in contact with the cation exchange membrane; the electrode plate located inside the electrolytic chamber is also provided to be in contact with the cation exchange membrane; and the electrode plate located outside the electrolytic chamber is surrounded by a side chamber. The electrolytic chamber was filled with 1.4 L of Fujisawa city tap water, and electrolysis was performed with an electrolytic current of 30 A by applying a direct-current voltage from a direct-current power source to both of the electrodes using the electrode plate provided inside the electrolytic chamber as the anode while using the electrode plate provided outside the electrolytic chamber and inside the side chamber as the cathode.

Concurrently with the start of electrolysis, a suction pump (vacuum pump, DAP-6D, available from ULVAC KIKO, Inc.) connected to the side chamber was operated to take in normal air with 6 L/min via an opening part of a diameter of 3 mm provided at the side chamber so that the normal air would be blown into the hydrogen gas generator. The hydrogen gas concentration in the side chamber and the electrolytic voltage were measured at a time when 1 minute elapsed after the start of electrolysis, at a time when 5 minutes elapsed after the start of electrolysis, and at a time when the electrolysis was completed (when 10 minutes elapsed after the start of electrolysis). Results thereof are listed in Table 1.

### [Comparative Example 1]

Without blowing normal air in Example 1, the hydrogen gas concentration in the side chamber and the electrolytic voltage were measured at a time when 10 seconds elapsed after the start of electrolysis and a time when 50 seconds elapsed after the start of electrolysis. Results thereof are also listed in Table 1 (the measurement interval was changed because the hydrogen concentration increased rapidly after the start of measurement).

**[Table 1]**

| | | After 1 minute | After 5 minutes | After 10 minutes |
|---|---|---|---|---|
| Example 1 | Hydrogen gas concentration (%) | 3,7 | 3,8 | 3,8 |
| | Electrolytic voltage (V) | 12,3 | 11,7 | 11,1 |
| | | After 10 seconds | After 50 seconds | |
| Comparative Example 1 | Hydrogen gas concentration (%) | 20,0 | 50 | |
| | Electrolytic voltage (V) | 10,1 | 10,1 | |

### [Description of Reference Numerals]

1... Apparatus for supplying hydrogen gas for a living organism
2... Subject raw water
3... Electrolytic chamber
4, 5... Electrode plate
6... Electrolytic cell
7... Gas phase portion
8... Hydrogen gas permeable membrane
9... Opening part
10... Diluent gas supplier
11... Suction pump
12, 16... Conduit pipe
13... Membrane
14... Direct-current power source
15... Spacer
16... Side chamber

## Claims

1. An apparatus for supplying hydrogen gas for a living organism comprising:
an electrolytic cell having an electrolytic chamber to which subject raw water to be electrolyzed is introduced, at least one membrane that partitions inside and outside of the electrolytic chamber, and at least a pair of electrode plates provided in the inside and the outside of the electrolytic chamber so as to sandwich the membrane, the electrode plate in the outside of the electrolytic chamber being provided to be in contact with the membrane;
a direct-current power source that applies a direct-current voltage to the pair of electrode plates; and
at least one of a diluent gas supplier or a vacuum pump for diluting hydrogen gas generated from the electrode plate that is to be a cathode,
wherein a part or whole of a gas phase portion in the electrolytic chamber is covered with a hydrogen gas permeable membrane,
the apparatus blows diluent gas supplied using the diluent gas supplier or the vacuum pump to the cathode or a cathode water surface thereby to constantly maintain a hydrogen gas concentration in a position separated by 7 cm from the cathode or the cathode water surface at lower than 18.3 vol% during electrolysis so that mixed gas comprising the hydrogen gas and the diluent gas is supplied to a living organism, the mixed gas having a hydrogen gas concentration of 0.1 vol% or higher and lower than 18.3 vol%.

2. The apparatus for supplying hydrogen gas for a living organism according to claim 1, wherein the electrode plate in the inside of the electrolytic chamber is provided to be in contact with the membrane.

3. The apparatus for supplying hydrogen gas for a living organism according to claim 1, wherein a side chamber is provided outside the electrolytic chamber, the side chamber enclosing one electrode plate of the pair of electrode plates.

4. The apparatus for supplying hydrogen gas for a living organism according to claim 3, wherein the subject raw water is introduced in the inside of the electrolytic chamber and in the side chamber.

5. The apparatus for supplying hydrogen gas for a living organism according to claim 1, wherein, in a state in which the subject raw water is introduced in the inside of the electrolytic chamber, the direct-current power source applies the direct-current voltage to both of the electrode plates using the electrode plate provided inside the electrolytic chamber as the cathode and the electrode plate provided outside the electrolytic chamber as an anode.

6. The apparatus for supplying hydrogen gas for a living organism according to claim 1, wherein the diluent gas is normal air.

7. The apparatus for supplying hydrogen gas for a living organism according to claim 1, wherein the diluent gas is blown at a flow volume of 0.5 L/min or more.

8. The apparatus for supplying hydrogen gas for a living organism according to claim 1, wherein a chlorine gas concentration in the mixed gas is 1 ppm or less.
